# EUROPEAN PATENT SPECIFICATION

(11) **EP 2 143 725 B1**
(45) Date of publication and mention of the grant of the patent: **12.08.2015**
(21) Application number: 08722373.1
(22) Date of filing: 18.03.2008
(51) Int. Cl.: C07H 15/04

(54) **METHOD FOR PRODUCTION OF LACTOSYLCERAMIDE**
VERFAHREN ZUR HERSTELLUNG VON LACTOSYLCERAMID
PROCÉDÉ DE FABRICATION DE LACTOSYLCÉRAMIDE

(30) Priority: 27.03.2007 JP 2007081009
(43) Date of publication of application: 13.01.2010
(73) Proprietor: MEGMILK SNOW BRAND Co., Ltd., Higashi-ku Sapporo (JP)
(72) Inventor: ISOGAI, Tomoyuki, Kawagoe-shi Saitama 350-1165 (JP); MIURA, Susumu, Tokyo 160-8575 (JP); KATO, Ken, Kawagoe-shi Saitama 350-1165 (JP); TANAKA, Reo, Kawagoe-shi Saitama 350-1165 (JP); MURAKAMI, Mototake, Tokyo 160-8575 (JP); YOSHIOKA, Toshimitsu, Kawagoe-shi Saitama 350-1165 (JP)
(74) Representative: f & e patent
(86) International application number: PCT/JP2008/054979
(87) International publication number: WO 2008/123070

(56) References cited:
- EP-A2- 0 133 170
- WO-A1-99/33475
- WO-A1-2006/025443
- JP-A- 03 099 091
- JP-A- 04 099 494
- JP-A- 05 279 379
- JP-A- 62 138 497
- JP-A- 2002 255 824

## Description

### Technical Field

The present invention relates to a method of manufacturing a lactosylceramide by heating ganglioside GD₃ and/or ganglioside GM₃ to be converted into a lactosylceramide, as well as a method of manufacturing a highly concentrated lactosylceramide by converting ganglioside GD₃ and/or ganglioside GM₃ in a milk-derived material into a lactosylceramide, and a highly concentrated lactosylceramide thus obtained.

A lactosylceramide or highly concentrated lactosylceramide obtained according to a manufacturing method conforming to the present invention is useful as a reagent in the fields of medicine, pharmacology, biochemistry, etc., and it is also useful as a material for producing pharmaceutical preparations, cosmetics, drinks and food, feeds, and so on.

### Prior Art

Lactosylceramide is a sphingoglycolipid constituted by a ceramide having the reducing end of lactose. Lactosylceramide is present in animal tissues, and it is known to be present at higher contents particularly in nerve organs. This lactosylceramide is also known as a common precursor for the globoside group, ganglioside and blood group active glycolipid group.

In addition, various physiological functions of lactosylceramide have been reported, and some reports suggest that lactosylceramide shows specific binding with Helicobacter pylori and is a component essential to differentiation of osteoclasts and maturation of neutrophils (Non-patent Literatures 1 to 3).

On the other hand, ganglioside GM₃ is structured in such a way that it is constituted by a ceramide bonding with the reducing end of lactose, where the non-reducing end of lactose is α 2-3 bonded with sialic acid. Ganglioside GD₃ has a structure whereby the non-reducing end of ganglioside GM₃ is further α 2-8 bonded with sialic acid, meaning that the entire ganglioside GD₃ molecule contains two sialic acid molecules.

In general, a representative method of manufacturing a lactosylceramide has been one where acetic acid, trichloroacetic acid or other acid is used to precipitate a glycolipid which is forming a complex together with a protein, and then the obtained precipitate is mixed with a chloroform-methanol mixture solution or other organic solvent containing alcohol to separate the glycolipid from the protein complex and thereby extract the glycolipid. However, such method cannot produce a composition with a relatively higher content. As a result, the refinement efficiency becomes poor and lactosylceramide cannot be obtained in an economical manner.

Another method of manufacturing a lactosylceramide has been proposed, where a protein hydrolyzing enzyme is added to a substance containing a neutral sphingoglycolipid derived from cow milk, etc., so that the enzyme acts upon this substance to produce a refined lactosylceramide. However, this method requires a complicated refinement process and therefore the manufacturing cost becomes invariably higher (Patent Literature 1). Other methods of manufacturing lactosylceramide are disclosed in JP 03 099091 and JP 05 279379.

As described above, conventional methods of manufacturing a lactosylceramide are time-consuming, and no method has been developed yet that allows for manufacturing of a highly concentrated lactosylceramide in an economical manner.
Patent Literature 1: Japanese Patent Laid-open No. Hei 2-169596
Non-patent Literature 1: J. Phar. Macol. Sci. 2006 Vol. 100, No. 3, pp. 195-200
Non-patent Literature 2: J. Biochem. Vol. 77, No. 8, p. 872
Non-patent Literature 3: Glycobiology. 1998 Apr; 8 (4): 297-309

### Summary of the Invention

### Problems to Be Solved by the Invention

As explained above, conventional methods require a complicated refinement process to obtain a highly concentrated lactosylceramide, and no method has been established yet that allows for refinement of a lactosylceramide in an efficient manner.

In light of the above, the present invention seeks to solve the problems of the aforementioned prior arts and it is an object of the present invention to provide a simple method of manufacturing a highly concentrated lactosylceramide efficiently and at relatively low cost. It is a further object of the present invention to provide a composition that contains a highly concentrated lactosylceramide obtained by such manufacturing method.

### Means for Solving the Problems

The inventors of the present invention studied in earnest in search of a method of manufacturing a highly concentrated lactosylceramide at relatively low cost in an easy, efficient and economical manner, and consequently found that ganglioside GD₃ and/or ganglioside GM₃ contained in a material could be converted into a lactosylceramide lactosylceramide.

Based on the above, the present invention encompasses the following constitutions:
(1) A method of manufacturing a lactosylceramide, **characterized in that** ganglioside GD₃ and/or ganglioside GM₃ is converted into a lactosylceramide by heating under an acidic condition. wherein the heating is performed in a pH range of 3 to 5 and temperature range of 70°C to 100°C for a period of 30 to 180 minutes.
(2) A method of manufacturing a lactosylceramide according to (1), characterized in manufacturing a highly concentrated lactosylceramide, wherein ganglioside GD₃ and/or ganglioside GM₃ in a milk-derived material is converted into a lactosylceramide wherein highly concentrated lactosylceramide refers to a lactosylceramide content in the solid as high as 0,01 to 8 wt.-%.
(3) A method of manufacturing a highly concentrated lactosylceramide according to (2), characterized in that the milk-derived material is constituted by one type or two or more types of materials selected from the group that consists of whey protein concentrate, butter milk, butter serum, skim milk and materials having high lipid fraction contents obtained from the foregoing materials using organic solvent.
(4) A method of manufacturing a highly concentrated lactosylceramide according to (2) or (3), characterized in that, after the heating in a pH range of 3 to 5 and temperature range of 70°C to 100°C for a period of 30 to 180 minutes, the pH is adjusted to a range of 4.5 to 5.0, followed by concentration using a method selected from that using either ultrafiltration membranes or microfiltration membranes.

### Effects of the Invention

A manufacturing method conforming to the present invention allows for manufacturing of a lactosylceramide easily and at relatively low cost from ganglioside GD₃ and/or ganglioside GM₃. In particular, a method conforming to the present invention allows a lactosylceramide to be manufactured from a milk or milk product containing a relatively large amount of protein, and therefore it is an effective method of manufacturing a highly concentrated lactosylceramide which is very useful in industrial settings.

A lactosylceramide or highly concentrated lactosylceramide manufactured by a manufacturing method conforming to the present invention has an infection protection function and other physiological functions and is therefore useful as a material for pharmaceutical preparations, cosmetics, drinks and food, feeds, etc., and the high concentration of lactosylceramide in the obtained composition is expected to widen the scope of application of the present invention.

### Best Mode for Carrying Out the Invention

One characteristic of a manufacturing method conforming to the present invention is that ganglioside GD₃ and/or ganglioside GM₃ is converted into a lactosylceramide by means of heating under an acidic condition, according to claim 1.

In other words, under the present invention ganglioside GD₃ and/or ganglioside GM₃ is heated under an acidic condition to desialize one sialic acid molecule bonding at the non-reducing end of the sialic acid section of ganglioside GD₃ and also desialize one sialic acid molecule bonding at the non-reducing end of the lactose constituting ganglioside GM₃, thereby converting ganglioside GD₃ or ganglioside GM₃ into a lactosylceramide in an efficient manner.

As for the material used in the aforementioned manufacturing method, not only ganglioside GD₃ and ganglioside GM₃, but a material that contains a large amount of whole milk, skim milk, or other protein and not much ganglioside GD₃ and/or ganglioside GM₃ can also be used. However, the material is preferably a milk-derived material, particularly a whey protein concentrate, butter milk, butter serum, skim milk or other milk-derived material containing a relatively large amount of ganglioside GD₃ and/or ganglioside GM₃.

Other materials that can be used include one prepared by a method where a material containing ganglioside GD₃ and/or ganglioside GM₃ is extracted using ethanol or other organic solvent (Japanese Patent Laid-open No. Hei 2-207090).

Under a manufacturing method conforming to the present invention, the pH and temperature settings during heating are very important. The lower the pH or higher the temperature, the higher the speed of production of lactosylceramide becomes, but the speed at which the lactosylceramide breaks down also increases. To manufacture a lactosylceramide at a high yield, therefore under an acidic condition the pH is adjusted to a range of 3 to 5, for example, preferably to a range of 3 to 4, while the temperature should be adjusted to a range of 70°C to 100°C.

Then, in consideration of the speed of production and speed of breakdown, a desired heating time can be set as deemed appropriate, according to the aforementioned heating condition, in a range of 30 to 180 minutes.

Also note that, if a milk-derived material is used, heating the material in a low-pH condition, such as at a pH below 3, will cause the supernatant to be mixed into the precipitate when casein, which is the key protein, is precipitated and removed, and consequently the precipitate cannot be separated from the supernatant completely and this may reduce the yield of the fraction containing lactosylceramide. This is because a pH of less than 3 prevents smooth precipitation of casein due to the fact that casein has a small grain size and is a stable protein. This results in an incomplete precipitation where the precipitate contains supernatant.

To increase the lactosylceramide concentration in the target composition, if a milk-derived material is used, it is preferable that the material is adjusted, after heating, to a pH range of 4.5 to 5.0 in which the isoelectric point of casein exists, in order to increase the grain size of casein and make casein an unstable protein which is easier to cohere and precipitate, and then remove such casein. It is also preferable that calcium chloride, etc., be added by 0.01 to 0.1 % to further increase the cohesive property of casein.

The casein precipitate obtained as mentioned above can be removed using a centrifugal separator, quark separator, nozzle separator, filter press or filtration cloth, through stationary standing, or by any other general removal method.

In addition, membrane filtration using ultrafiltration membranes, microfiltration membranes, dialysis membranes, electrodialysis membranes, etc., is effective in increasing the concentration of the target fraction containing lactosylceramide. In this case, preferably, membrane filtration uses microfiltration (MF) membranes with a pore diameter of 0.1 to 2.0 µm or ultrafiltration (UF) membranes with a molecular weight cut off of 5 to 500 kDa.

In the above, the concentrated liquid material can be neutralized using potassium hydroxide, potassium chloride or any other general neutralizing agent.

The obtained concentrated liquid material containing lactosylceramide can be concentrated, dried and solidified using any appropriate method such as vacuum drying or freeze drying, and through this method a highly concentrated lactosylceramide can be obtained where the lactosylceramide content in the total solid is as high as 0.01 to 8 percent by weight. The lactosylceramide content was measured by means of HPLC.

Next, examples are given to explain the present invention in detail.

It should be noted, however, that the examples described below are given solely for the purpose of explaining the present invention and the present invention is not at all limited to what is described in these examples.

### Example 1

A ganglioside GM₃ composition was prepared according to a ganglioside GM₃ composition and a method of manufacturing the same (Japanese Patent Laid-open No. Hei 5-279379), and then 500 mg of this ganglioside GM₃ composition was dissolved in 2 liters of water, after which 15% hydrochloric acid was added to adjust the pH to 3.0 and the pH-adjusted mixture was heated at 88°C for 180 minutes. Next, the supernatant was neutralized with 10% potassium hydroxide and then freeze dried to obtain 372 mg of white powder.

According to a quantification using HPLC, this white powder contained 99 percent by weight or more of lactosylceramide.

### Example 2

15% hydrochloric acid was added to a 10% (by weight) aqueous butter serum solution containing 230 mg of ganglioside GD₃ per liter to adjust the pH to 4.5, while at the same time calcium chloride was added to a final content of 0.03 percent by weight of the total volume, after which the mixture was heated at 88°C for 180 minutes. Next, the produced casein precipitate was completely removed using a centrifugal separator to obtain a supernatant.

This supernatant was neutralized with 10% potassium hydroxide and then the neutralized mixture was freeze dried to obtain a highly concentrated lactosylceramide powder conforming to the present invention.

When the lipid content in the obtained powder was measured by the Roese-Gottlieb method, the powder contained 16 percent by weight of lipid.

According to a quantification using HPLC, this composition contained 0.5 percent by weight of lactosylceramide.

### Example 3

15% hydrochloric acid was added to a 10% (by weight) aqueous butter serum solution containing 230 mg of ganglioside GD₃ per liter to adjust the pH to 4.5, while at the same time calcium chloride was added to a final content of 0.03 percent by weight of the total volume, after which the mixture was heated at 88°C for 180 minutes. Next, the produced casein precipitate was completely removed using a centrifugal separator to obtain a supernatant.

This supernatant was neutralized with 10% potassium hydroxide and then the neutralized mixture was filtered using microfiltration membranes with a fraction grain size of 0.1 µM (by Millipore Corporation) to obtain a concentrated liquid material.

Then, this concentrated liquid material was freeze dried to obtain a highly concentrated lactosylceramide powder conforming to the present invention.

When the lipid content in the obtained powder was measured by the Roese-Gottlieb method, the powder contained 60 percent by weight of lipid.

According to a quantification using HPLC, this composition contained 1 percent by weight of lactosylceramide.

### Example 4

15% hydrochloric acid was added to a 10% (by weight) aqueous butter serum solution containing 230 mg of ganglioside GD₃ per liter to adjust the pH to 3.5, while at the same time calcium chloride was added to a final content of 0.03 percent by weight of the total volume, after which the mixture was heated at 88°C for 180 minutes. Next, the pH was adjusted to a range of 4.5 to 5.0 to cohere casein, which was the main protein in the material. The produced casein precipitate was then completely removed using a centrifugal separator to obtain a supernatant.

This supernatant was neutralized with 10% potassium hydroxide and then the neutralized mixture was filtered using microfiltration membranes with a fraction grain size of 0.1 µM (by Millipore Corporation) to obtain a concentrated liquid material. Then, this concentrated liquid material was freeze dried to obtain a highly concentrated lactosylceramide powder conforming to the present invention.

When the lipid content in the obtained powder was measured by the Roese-Gottlieb method, the powder contained 60 percent by weight of lipid.

According to a quantification using HPLC, this composition contained 1.5 percent by weight of lactosylceramide.

### Example 5

15% hydrochloric acid was added to a 10% (by weight) aqueous butter serum solution containing 230 mg of ganglioside GD₃ per liter to adjust the pH to 3.0, while at the same time calcium chloride was added to a final content of 0.03 percent by weight of the total volume, after which the mixture was heated at 88°C for 180 minutes. Next, the pH was adjusted to a range of 4.5 to 5.0 to cohere casein, which was the main protein in the material. The produced casein precipitate was then completely removed using a centrifugal separator (by Beckman Coulter, Inc.) to obtain a supernatant.

This supernatant was neutralized with 10% potassium hydroxide and then the neutralized mixture was filtered using microfiltration membranes with a fraction grain size of 0.1 µM (by Millipore Corporation) to obtain a concentrated liquid material. Then, this concentrated liquid material was freeze dried to obtain a highly concentrated lactosylceramide powder conforming to the present invention.

When the lipid content in the obtained powder was measured by the Roese-Gottlieb method, the powder contained 60 percent by weight of lipid.

According to a quantification using HPLC, this composition contained 3.6 percent by weight of lactosylceramide.

### Example 6

15% hydrochloric acid was added to a 10% (by weight) aqueous butter serum solution containing 230 mg of ganglioside GD₃ per liter to adjust the pH to 3.0, while at the same time calcium chloride was added to a final content of 0.03 percent by weight of the total volume, after which the mixture was heated at 88°C for 180 minutes. Next, the pH was adjusted to a range of 4.5 to 5.0 to cohere casein, which was the main protein in the material. The produced casein precipitate was then completely removed using a centrifugal separator to obtain a supernatant.

This supernatant was neutralized with 10% potassium hydroxide and then freeze dried to obtain a highly concentrated lactosylceramide powder conforming to the present invention.

When the lipid content in the obtained powder was measured by the Roese-Gottlieb method, the powder contained 16 percent by weight of lipid.

According to a quantification using HPLC, this composition contained 0.9 percent by weight of lactosylceramide.

### Example 7

15% hydrochloric acid was added to a 10% (by weight) aqueous butter serum solution containing 200 mg of ganglioside GD₃ per liter to adjust the pH to 3.0, while at the same time calcium chloride was added to a final content of 0.03 percent by weight of the total volume, after which the mixture was heated at 80°C for 180 minutes. Next, the pH was adjusted to a range of 4.5 to 5.0 to cohere casein, which was the main protein in the material. The produced casein precipitate was then completely removed using a centrifugal separator to obtain a supernatant.

This supernatant was neutralized with 10% potassium hydroxide and then the neutralized mixture was filtered using microfiltration membranes with a fraction grain size of 0.1 µM (by Millipore Corporation) to obtain a concentrated liquid material. Then, this concentrated liquid material was freeze dried to obtain a highly concentrated lactosylceramide powder conforming to the present invention.

When the lipid content in the obtained powder was measured by the Roese-Gottlieb method, the powder contained 60 percent by weight of lipid.

According to a quantification using HPLC, this composition contained 3 percent by weight of lactosylceramide.

### Example 8

A ganglioside material containing 360 mg of ganglioside GM₃ per liter was prepared from butter serum according to a method of manufacturing ganglioside GM₃ through acid hydrolysis of ganglioside GD₃ (Japanese Patent Laid-open No. Hei 5-279379). Thereafter, casein, which was the main protein in the material, was cohered and the produced casein precipitate was completely removed using a centrifugal separator to obtain a supernatant.

This supernatant was neutralized with 10% potassium hydroxide and then the neutralized mixture was filtered using microfiltration membranes with a fraction grain size of 0.1 µM (by Millipore Corporation) to obtain a concentrated liquid material. Then, this concentrated liquid material was freeze dried to obtain a powder. The obtained ganglioside material powder was made into a 10% (by weight) aqueous solution, to which 15% hydrochloric acid was added to adjust the pH to 3.0, and then the pH-adjusted solution was heated at 88°C for 180 minutes.

Thereafter, the heated solution was neutralized with 10% potassium hydroxide and then freeze dried to obtain a highly concentrated lactosylceramide powder conforming to the present invention.

When the lipid content in the obtained powder was measured by the Roese-Gottlieb method, the powder contained 60 percent by weight of lipid.

According to a quantification using HPLC, this composition contained 4.8 percent by weight of lactosylceramide.

### Example 9

15% hydrochloric acid was added to 5 liters of a skim milk containing 3.8 mg of ganglioside GD₃ per liter and 0.1 mg of ganglioside GM₃ per liter to adjust the pH to 3.0, while at the same time calcium chloride was added to a final content of 0.03 percent by weight of the total volume, after which the mixture was heated at 88°C for 180 minutes. Next, the pH was adjusted to a range of 4.5 to 5.0 to cohere casein, which was the main protein in the material. The produced casein precipitate was then completely removed using a centrifugal separator to obtain a supernatant.

This supernatant was neutralized with 10% potassium hydroxide and then freeze dried to obtain a highly concentrated lactosylceramide powder conforming to the present invention.

When the lipid content in the obtained powder was measured by the Roese-Gottlieb method, the powder contained 0.1 percent by weight of lipid.

According to a quantification using HPLC, this composition contained 0.01 percent by weight of lactosylceramide.

### Example 10

15% hydrochloric acid was added to a 10% (by weight) butter milk solution containing 90 mg of ganglioside GD₃ per liter to adjust the pH to 3.0, while at the same time calcium chloride was added to a final content of 0.03 percent by weight of the total volume, after which the mixture was heated at 88°C for 180 minutes. Next, the pH was adjusted to a range of 4.5 to 5.0 to cohere casein, which was the main protein in the material. The produced casein precipitate was then completely removed using a centrifugal separator to obtain a supernatant.

This supernatant was neutralized with 10% potassium hydroxide and then freeze dried to obtain a highly concentrated lactosylceramide powder conforming to the present invention.

When the lipid content in the obtained powder was measured by the Roese-Gottlieb method, the powder contained 7 percent by weight of lipid.

According to a quantification using HPLC, this composition contained 0.2 percent by weight of lactosylceramide.

### Example 11

15% hydrochloric acid was added to a 10% (by weight) liquid whey protein concentrate (WPC solution) containing 130 mg of ganglioside GD₃ per liter to adjust the pH to 3.0, after which the mixture was heated at 88°C for 180 minutes.

This supernatant was neutralized with 10% potassium hydroxide and then freeze dried to obtain a highly concentrated lactosylceramide powder conforming to the present invention.

When the lipid content in the obtained powder was measured by the Roese-Gottlieb method, the powder contained 10 percent by weight of lipid.

According to a quantification using HPLC, this composition contained 0.3 percent by weight of lactosylceramide.

## Claims

1. A method of manufacturing a lactosylceramide, **characterized in that** ganglioside GD₃ and/or ganglioside GM₃ is converted into a lactosylceramide by heating under an acidic condition, wherein the heating is performed in a pH range of 3 to 5 and temperature range of 70°C to 100°C for a period of 30 to 180 minutes.

2. A method of manufacturing a lactosylceramide according to claim 1,
**characterized in** manufacturing a highly concentrated lactosylceramide, wherein ganglioside GD₃ and/or ganglioside GM₃ in a milk-derived material is converted into a lactosylceramidewherein highly concentrated lactosylceramide refers to a lactosylceramide content in the solid as high as 0,01 to 8 percent by weight.

3. The method of manufacturing a highly concentrated lactosylceramide according to Claim 2, **characterized in that** the milk-derived material is constituted by one type or two or more types of materials selected from the group consisting of whey protein concentrate, butter milk, butter serum, skim milk, and materials having high lipid fraction contents obtained from the foregoing materials using organic solvent.

4. The method of manufacturing a highly concentrated lactosylceramide according to Claim 2 or 3, **characterized in that**, after the heating in a pH range of 3 to 5 and temperature range of 70°C to 100°C for a period of 30 to 180 minutes, the pH is adjusted to a range of 4.5 to 5.0, followed by concentration using a method selected from that using either ultrafiltration membranes or microfiltration membranes.

## Patentansprüche

1. Ein Verfahren zur Herstellung eines Lactosylceramids, **dadurch gekennzeichnet, dass** Gangliosid GD₃ und/oder Gangliosid GM₃ durch Erwärmen unter sauren Bedingungen in ein Lactosylceramid umgewandelt wird, wobei das Erwärmen in einem pH-Bereich von 3 bis 5 und einem Temperaturbereich von 70°C bis 100°C für eine Zeitdauer von 30 bis 180 Minuten durchgeführt wird.

2. Ein Verfahren zum Herstellen eines Lactosylceramids gemäß Anspruch 1, **gekennzeichnet durch** das Herstellen eines hochkonzentrierten Lactosylceramids, wobei Gangliosid GD₃ und/oder Gangliosid GM₃ in einem aus Milch erhaltenen Material in ein Lactosylceramid umgewandelt wird, wobei hochkonzentriertes Lactosylceramid sich auf einen Lactosylceramid-Gehalt im Feststoff in einer Höhe von 0,01 bis 8 Gewichtsprozent bezieht.

3. Das Verfahren zur Herstellung eines hochkonzentrierten Lactosylceramids gemäß Anspruch 2, **dadurch gekennzeichnet, dass** das aus Milch erhaltene Material aus einer Art oder zwei oder mehr Arten von Materialien besteht, ausgewählt aus der Gruppe, bestehend aus Molkeproteinkonzentrat, Buttermilch, Butterserum, entrahmter Milch und Materialien mit hohen Gehalten des Lipidanteils, die aus den vorhergehenden Materialien unter Verwendung eines organischen Lösemittels erhalten werden.

4. Das Verfahren zur Herstellung eines hochkonzentrierten Lactosylceramids gemäß Anspruch 2 oder 3, **dadurch gekennzeichnet, dass** nach dem Erwärmen in einem pH-Bereich von 3 bis 5 und einem Temperaturbereich von 70°C bis 100°C für eine Zeitdauer von 30 bis 180 Minuten der pH in einem Bereich von 4.5 bis 5.0 eingestellt wird, gefolgt von einer Aufkonzentrierung unter Anwendung eines Verfahrens, ausgewählt aus einem, das entweder Ultrafiltrationsmembran oder Mikrofiltrationsmembranen verwendet.

## Revendications

1. Procédé de fabrication d'un lactosylcéramide, **caractérisé en ce que** le ganglioside GD₃ et/ou le ganglioside GM₃ sont convertis en un lactosylcéramide par chauffage dans une condition acide, dans lequel le chauffage est réalisé dans une plage de pH de 3 à 5 et dans une plage de températures de 70°C à 100°C pendant une durée de 30 à 180 minutes.

2. Procédé de fabrication d'un lactosylcéramide selon la revendication 1, **caractérisé par** la fabrication d'un lactosylcéramide hautement concentré, dans lequel le ganglioside GD₃ et/ou le ganglioside GM₃ dans un matériau dérivé du lait sont convertis en un lactosylcéramide, dans lequel lactosylcéramide hautement concentré fait référence à une teneur en lactosylcéramide dans le solide pouvant atteindre jusqu'à 0,01 à 8 % en poids.

3. Procédé de fabrication d'un lactosylcéramide hautement concentré selon la revendication 2, **caractérisé en ce que** le matériau dérivé du lait est constitué d'un type ou de deux types ou plus de matériaux sélectionnés dans le groupe constitué d'un concentré de protéine de lactosérum, du babeurre, du sérum de beurre, du lait écrémé, et de matériaux ayant des teneurs élevées en fraction lipidique obtenus des matériaux précédents en utilisant un solvant organique.

4. Procédé de fabrication d'un lactosylcéramide hautement concentré selon la revendication 2 ou 3, **caractérisé en ce que**, après le chauffage dans une plage de pH de 3 à 5 et dans une plage de températures de 70°C à 100°C pendant une durée de 30 à 180 minutes, le pH est ajusté à une plage de 4,5 à 5,0, suivi par la concentration en utilisant un procédé sélectionné parmi un procédé utilisant des membranes d'ultrafiltration ou un procédé utilisant des membranes de microfiltration.
